# EUROPEAN PATENT SPECIFICATION

(11) **EP 2 773 319 B1**
(45) Date of publication and mention of the grant of the patent: **14.03.2018**
(21) Application number: 12783840.7
(22) Date of filing: 02.11.2012
(51) Int. Cl.: A61K 8/65, A61K 8/73, A61Q 19/00, A61Q 19/08, A61L 27/52

(54) **HYALURONIC ACID-COLLAGEN MATRICES FOR DERMAL FILLING AND VOLUMIZING APPLICATIONS**
HYALURONSÄURE-COLLAGEN MATRIZEN ZUR DERMALEN FÜLLUNG UND VOLUMENVERGRÖSSERUNG
MATRICES D'ACIDE HYALURONIQUE ET DU COLLAGENE POUR UN REMPLISSAGE DERMIQUE ET D'AUGMENTATION DU VOLUME

(30) Priority: 04.11.2011 US 201161555970 P; 04.09.2012 US 201213603213; 06.09.2012 US 201213605565
(43) Date of publication of application: 10.09.2014
(73) Proprietor: ALLERGAN, INC., Irvine, CA 92612 (US)
(72) Inventor: YU, Xiaojie, Irvine, CA 92602 (US); MANESIS, Nicholas, J., Summerland, CA 93067 (US); POLLOCK, Jacob, F., Charleston, WV 25314 (US)
(74) Representative: Hoffmann Eitle
(86) International application number: PCT/US2012/063228
(87) International publication number: WO 2013/067293

(56) References cited:
- EP-A1- 1 419 792
- WO-A1-2010/053918
- WO-A1-2011/119468
- WO-A2-2010/003104
- PARK S-N ET AL: "Characterization of porous collagen/hyaluronic acid scaffold modified by 1-ethyl-3-(3-dimethylaminopropyl)carbodiim ide cross-linking", BIOMATERIALS, ELSEVIER SCIENCE PUBLISHERS BV., BARKING, GB, vol. 23, no. 4, 15 February 2002 (2002-02-15), pages 1205-1212, XP004348139, ISSN: 0142-9612, DOI: 10.1016/S0142-9612(01)00235-6
- PARK S ET AL: "Biological characterization of EDC-crosslinked collagen-hyaluronic acid matrix in dermal tissue restoration", BIOMATERIALS, ELSEVIER SCIENCE PUBLISHERS BV., BARKING, GB, vol. 24, no. 9, 1 April 2003 (2003-04-01), pages 1631-1641, XP004404219, ISSN: 0142-9612, DOI: 10.1016/S0142-9612(02)00550-1

## Description

This application generally relates to biocompatible, implantable compositions and more specifically relates to hyaluronic acid-collagen based compositions useful as dermal fillers.

### SUMMARY

The present invention generally relates to a soft tissue aesthetic product. In one aspect, the product comprises a filler comprising a hydrogel having a form suitable for injecting into human tissue; and a label comprising instructions to inject the filler into the human tissue; wherein the hydrogel comprises water, and a crosslinked macromolecular matrix described herein.

Some embodiments include a method of improving an aesthetic quality of soft tissue of a human being comprising: injecting a hydrogel composition into a soft tissue of the human being to thereby improve the aesthetic quality of the soft tissue; wherein the hydrogel composition comprises water, and a crosslinked macromolecular matrix described herein.

Some embodiments include a method of generating tissue comprising contacting a tissue with a hydrogel composition to generate an additional amount of the tissue, wherein the hydrogel composition comprises water and a crosslinked macromolecular matrix described herein.

Some crosslinked molecular matrices may comprise a hyaluronic acid component; and a collagen component; wherein the hyaluronic acid component is crosslinked to the collagen component by a crosslinking component; and wherein the crosslinking component comprises a plurality of crosslink units, wherein at least a portion of the crosslink units comprise an ester bond or an amide bond.

EP 1 419 792 discloses a glycosaminoglycan-polycation complex as a matrix material for use in tissue regeneration, such as cartilage repair, and a preparation method thereof. The complex is synthesized by crosslinking glycosaminoglycan and polycation with a polyfunctional crosslinking agent under physiological conditions. In this method, the crosslinking agent consists of polyethyleneglycol having two or more electrophilic leaving groups (e.g. succinimidyl group or its derivatives) at the carboxyl terminal thereof, and the concentration of the crosslinking agent is in the range of 0.3 to 3 mM. According to this disclosure, the crosslinking reaction can be performed under physiological conditions to avoid conventional problems of adverse affect on cells (cell death) and the formation of a polyion complex (inhomogeneous precipitate) due to a crosslinking reaction in alcohol or water. It is stated that this crosslinking reaction can be conducted in the presence of cells mixed with the glycosaminoglycan and polycation in advance.

### BRIEF DESCRIPTION OF THE DRAWINGS

Some aspects of the present disclosure may be more clearly understood with reference to the appended drawings of which:
FIG.1 is a plot of frequency sweep and strain sweep for a hydrogel in accordance with this disclosure.
FIG. 2 is an extrusion profile through a 30G needle for a hydrogel from Example 4.

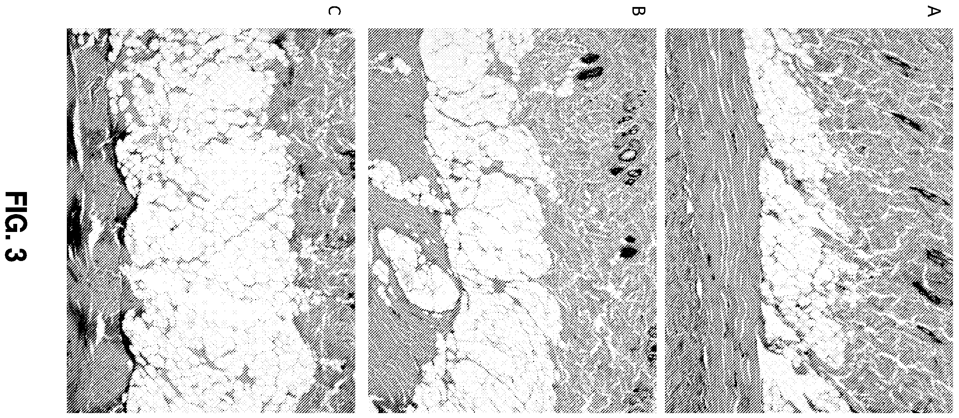

FIG. 3 shows micrographs (at 5X magnification) of (A) tissue adjacent to an implanted control composition of commercial crosslinked hyaluronic acid gel, (B) tissue adjacent to an implanted composition of Example 3, and (C) tissue adjacent to an implanted composition from Example 4.

### DETAILED DESCRIPTION

Some embodiments include a method of improving an aesthetic quality of soft tissue of a human. Such a method may comprise injecting a hydrogel composition, or a composition comprising a hydrogel, into a soft tissue of the human being to thereby improve the aesthetic quality of the soft tissue.

Some embodiments may include a soft tissue aesthetic product comprising: a filler comprising a hydrogel having a form suitable for injecting into human tissue and a label comprising instructions to inject the filler into the human tissue.

A filler comprising a hydrogel or a hydrogel composition may be any kind of filler that is suitable for injecting into human tissue to improve an aesthetic quality of a soft tissue, such as a dermal filler, a breast augmentation or reconstruction filler, a lip filler, hand rejuvenation, or the like. When injected, a hydrogel may stimulate tissue in-growth and generation after being injected into the soft tissue. In some embodiments, a hydrogel may stimulate collagenesis after being injected into the soft tissue.

Injecting a hydrogel comprising a crosslinked macromolecular matrix comprising a hyaluronic acid component that is crosslinked to a collagen component may provide improved aesthetic quality for an extended duration, as compared to injecting an identical hydrogel except that hyaluronic acid component and the collagen component are not crosslinked.

Some embodiments include a method of generating tissue comprising contacting a tissue with a hydrogel composition to generate an additional amount of the tissue. This method may be used to generate tissue both ex vivo and in vivo. In some embodiments, contact between a tissue and a hydrogel may be *ex vivo.* In some embodiments, contact between a tissue and a hydrogel may be *in vivo.* Tissue types that may be generated include, but are not limited to, adipose tissue, muscle tissue, tendon tissue, cardiovascular tissue, neural tissue, bone tissue, and the like.

Some embodiments include a packaged product comprising a syringe loaded with a hydrogel and a needle. A syringe may be fitted with a needle of any size that is appropriate for injecting the hydrogel into the soft tissue of interest, such as a needle with about a #25, about a #30, or a larger gauge.

A filler comprising a hydrogel may be suitable for injection if it can be injected into the soft tissue of interest without unreasonable difficulty, and includes fillers that can be dispensed from cannulas having gauge as low as about #30 or about #25 under normal manual pressure with a smooth extrusion plateau.

Injection of a hydrogel may provide a soft tissue augmentation that mimics the natural components of the skin. A hydrogel may be injected intradermally or subcutaneously to augment soft tissue and to repair or correct congenital anomalies, acquired defects, or cosmetic defects. Examples of such conditions include congenital anomalies such as hemifacial microsomia, malar and zygomatic hypoplasia, unilateral mammary hypoplasia, pectus excavatum, pectoralis agenesis (Poland's anomaly), and velopharyngeal incompetence secondary to cleft palate repair or submucous cleft palate (as a retropharyngeal implant); acquired defects (post traumatic, post surgical, or post infectious) such as depressed scars, subcutaneous atrophy (e.g., secondary to discoid lupis erythematosis), keratotic lesions, enopthalmos in the unucleated eye (also superior sulcus syndrome), acne pitting of the face, linear scleroderma with subcutaneous atrophy, saddle-nose deformity, Romberg's disease, and unilateral vocal cord paralysis; and cosmetic defects such as glabellar frown lines, deep nasolabial creases, circum-oral geographical wrinkles, sunken cheeks, and mammary hypoplasia.

Crosslinked hyaluronic acid, collagen, and crosslinked collagen, such as those used in dermal fillers, do not promote cellular infiltration and tissue in-growth. Similarly, collagen simply blended into hyaluronic acid hydrogels does not promote tissue integration or *de novo* tissue generation. However, some hydrogels described herein do promote cellular migration into the hydrogels and tissue formation within the gels when implanted *in vivo.*

A hydrogel may comprise water and a crosslinked macromolecular matrix. Typically, a crosslinked molecular matrix may comprise a hyaluronic acid component and a collagen component, wherein the hyaluronic acid component is crosslinked to the collagen component by a crosslinking component. A crosslinking component may comprise a plurality of crosslink units, wherein at least a portion of the crosslink units comprise an ester bond or an amide bond.

A crosslinked macromolecular matrix for a hydrogel may be synthesized by coupling a hyaluronic acid with a collagen using a coupling agent, such as a carbodiimide. In these hydrogels, hyaluronic acid may serve as a biocompatible water-binding component, providing bulk and isovolumetric degradation. Additionally, collagen may impart cell adhesion and signaling domains to promote cell attachment, migration, and other cell functions such as extra-cellular matrix deposition. The biopolymers form homogeneous hydrogels with tunable composition, swelling, and mechanical properties. Compositions can be made to be injectable for minimally invasive implantation through syringe and needle.

Hyaluronic acid is a non-sulfated glycosaminoglycan that enhances water retention and resists hydrostatic stresses. It is non-immunogenic and can be chemically modified in numerous fashions. Hyaluronic acid may be anionic at pH ranges around or above the pKa of its carboxylic acid groups.

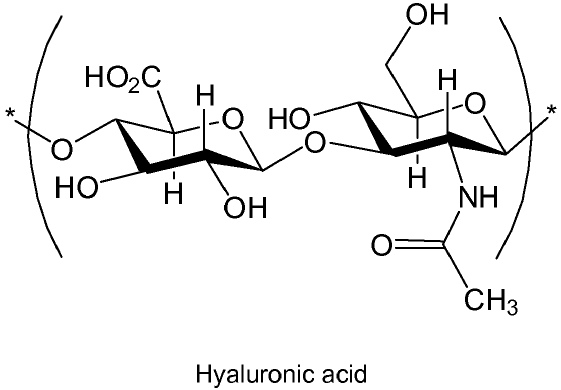

Collagen is a protein that forms fibrils and sheets that bear tensile loads. Collagen also has specific integrin-binding sites for cell adhesion and is known to promote cell attachment, migration, and proliferation. Collagen may be positively charged because of its high content of basic amino acid residues such as arginine, lysine, and hydroxylysine.

Because hyaluronic acid may be anionic and collagen may be cationic, the two macromolecules may form polyionic complexes in aqueous solution. A polyionic complex may be significantly less soluble in water than either hyaluronic acid or collagen, and thus may precipitate out of aqueous solution when the two macromolecules are together in a mixture.

Under certain conditions, a hyaluronic acid and a collagen may be combined in an aqueous liquid in which both components are soluble. A hyaluronic acid and a collagen may then be crosslinked while both are dissolved in an aqueous solution to form a hydrogel. Reaction conditions such as the concentration of hyaluronic acid, the concentration of collagen, the pH of the solution, and salt concentration may be adjusted to help to prevent polyionic complex formation between anionic hyaluronic acid and cationic collagen. They may also help to prevent collagen microfibril formation.

Some embodiments include a method of crosslinking hyaluronic acid and collagen. This method generally comprises a dissolution step which results in an aqueous pre-reaction solution. In a dissolution step, hyaluronic acid and collagen are dissolved in an aqueous solution that has a low pH and/or a salt to form an aqueous pre-reaction solution.

A hyaluronic acid-collagen crosslinking method further comprises an activation step. In an activation step, an aqueous pre-reaction solution is modified at least by adding a water soluble coupling agent and/or by increasing the pH of the solution. If needed, a salt may also be added to keep the hyaluronic acid and collagen in solution at the higher pH. Thus, a crosslinking reaction mixture comprises hyaluronic acid and collagen dissolved or dispersed in an aqueous medium, a water soluble coupling agent, and a salt, and has a higher pH than the aqueous pre-reaction solution from which it was derived. The crosslinking reaction mixture is allowed to react to thereby crosslink the hyaluronic acid and the collagen.

In some embodiments, the pH of the aqueous pre-reaction solution may be increased and a substantial amount of fiber formation may be allowed to occur in the solution before adding the water soluble coupling agent. In some embodiments, the water soluble coupling agent may be added to the aqueous pre-reaction solution before substantially any fiber formation occurs.

A crosslinking reaction mixture can react to form a crosslinked macromolecular matrix. Since reaction occurs in an aqueous solution, a crosslinked macromolecular matrix may be dispersed in an aqueous liquid in hydrogel form as it is formed by a crosslinking reaction. A crosslinked macromolecular matrix may be kept in hydrogel form because, in many instances, a crosslinked macromolecular matrix may be used in hydrogel form.

In some embodiments, an aqueous pre-reaction solution or a crosslinking reaction mixture may further comprise about 10% to about 90% of an organic solvent in which hyaluronic acid has poor solubility, such as ethanol, methanol, isopropanol, or the like.

After a crosslinking reaction has occurred, the crosslinked macromolecular matrix may be particulated or homogenized through a mesh. This may help to form an injectable slurry or hydrogel. A mesh used for particulating a crosslinked macromolecular matrix may have any suitable pore size depending upon the size of particles desired. In some embodiments, the mesh may have a pore size of about 10 microns to about 100 microns, about 50 microns to about 70 microns, or about 60 microns.

A hydrogel comprising a crosslinked molecular matrix may be treated by dialysis for sterilization or other purposes. Dialysis may be carried out by placing a semipermeable membrane between the hydrogel and another liquid so as to allow the hydrogel and the liquid to exchange molecules or salts that can pass between the membrane.

A dialysis membrane may have a molecular weight cutoff that may vary. For example, the cutoff may be about 5,000 daltons to about 100,0000 daltons, about 10,000 daltons to about 30,000 daltons, or about 20,000 daltons.

The dialysis may be carried out against a buffer solution, or the liquid on the other side of the membrane from the hydrogel may be a buffer solution. In some embodiments, the buffer solution may be a sterile phosphate buffer solution that may comprise phosphate buffer, potassium chloride, and/or sodium chloride. A sterile phosphate buffer solution may be substantially isosmotic with respect to human physiological fluid. Thus, when dialysis is complete, the liquid component of a hydrogel may be substantially isosmotic with respect to human physiological fluid.

In some embodiments, a crosslinked macromolecular complex may further comprise an aqueous liquid. For example, the crosslinked macromolecular complex may absorb the aqueous liquid so that a hydrogel is formed. An aqueous liquid may comprise water with a salt dissolved in it, such as a phosphate buffer, sodium chloride, potassium chloride, etc. In some embodiments, an aqueous liquid may comprise water, sodium chloride at a concentration of about 100 mM to about 200 mM, potassium chloride at a concentration of about 2 mM to about 3 mM, and phosphate buffer at a concentration of about 5 mM to about 15 mM, wherein the pH of the liquid is about 7 to about 8.

A hydrogel may be used in a soft tissue aesthetic product. A soft tissue aesthetic product may comprise: an aesthetic device having a form suitable for injecting or implanting into human tissue; and a label comprising instructions to inject or implant the aesthetic component into human tissue; wherein the aesthetic device comprises a crosslinked macromolecular matrix described herein. Some products may comprise the crosslinked macromolecular matrix in hydrogel form.

Some embodiments include a method of improving an aesthetic quality of an anatomic feature of a human being comprising: injecting or implanting an aesthetic device into a tissue of the human being to thereby improve the aesthetic quality of the anatomic feature; wherein the aesthetic device comprises a crosslinked macromolecular matrix comprising described herein. In some embodiments, the crosslinked macromolecular matrix used in the product may be in hydrogel form.

In some embodiments, a hydrogel of a crosslinked macromolecular complex may have a storage modulus of about 1 Pa to about 10,000 Pa, about 50 Pa to 10,000 Pa, about 500 Pa to about 1000 Pa, about 556 Pa, about 560 Pa, about 850 Pa, about 852 Pa, or any value in a range bounded by, or between, any of these values.

In some embodiments, a hydrogel of a crosslinked macromolecular complex may have a loss modulus of about 1 Pa to about 500 Pa, about 10 Pa to 200 Pa, about 100 Pa to about 200 Pa, about 20 Pa, about 131 Pa, about 152 Pa, or any value in a range bounded by, or between, any of these values.

In some embodiments, a hydrogel of a crosslinked macromolecular complex may have an average extrusion force of about 20 N to 30 N, or about 25 N, when the hydrogel is forced through a 30G needle syringe by moving the plunger of a 1 mL syringe containing the hydrogel at a rate of 100 mm/min for about 11 mm, and measuring the average force from about 4 mm to about 10 mm.

A crosslinked macromolecular complex may have tunable swelling properties based on reaction conditions and hydrogel dilution. In some embodiments, a crosslinked macromolecular complex may have a swelling ratio of about 1 to about 7. A swelling ratio is the ratio of the weight of the crosslinked macromolecular complex when saturated with water to the weight of the crosslinked macromolecular complex without any water. More specifically, the swelling ratio is the ratio of the mass of the gel which has been allowed to fully swell to the mass of the gel at its initial concentration.

In a crosslinking reaction, the molecular weight of a hyaluronic acid may vary. In some embodiments, a hyaluronic acid may have a molecular weight of about 500,000 daltons to about 10,000,000 daltons, about 1,000,000 daltons to about 5,000,000 daltons, or about 1,000,000 daltons to about 3,000,000 daltons. When the crosslinking reaction occurs, the resulting crosslinked macromolecular product may have a hyaluronic acid component derived from the hyaluronic acid in the crosslinking reaction. Thus, the ranges recited above may also apply to the molecular weight of a hyaluronic acid component, e.g. about 500,000 daltons to about 10,000,000 daltons, about 1,000,000 daltons to about 5,000,000 daltons, or about 1,000,000 daltons to about 3,000,000 daltons. The term "molecular weight" is applied in this situation to a portion of the matrix even though the hyaluronic acid component may not actually be a separate molecule due to the crosslinking.

The concentration of hyaluronic acid in an aqueous pre-reaction solution or a crosslinking reaction mixture may vary. In some embodiments, hyaluronic acid is present at about 3 mg/mL to about 100 mg/mL, about 6 mg/mL to about 24 mg/mL, about 1 mg/mL to about 30 mg/mL, about 1.7 mg/mL, about 3 mg/mL, about 6 mg/mL, about 12 mg/mL, about 16 mg/mL, or about 24 mg/mL

Any type of collagen may be used in the methods and compositions described herein. In some embodiments, collagen type I, collagen type III, collagen type IV, collagen type VI, or a combination thereof, may be used. In some embodiments, a collagen or a collagen component comprises collagen type I or collagen type III. In some embodiments, the collagen component comprises collagen type V.

A collagen may be derived from cell culture, animal tissue, or recombinant means, and may be derived from human, porcine, or bovine sources. Some embodiments comprise collagen derived from human fibroblast culture. Some embodiments comprise collagen that has been denatured to gelatin. The source and/or collagen extraction/processing conditions can alter the way in which collagen macromolecules bundle together to form supramolecular structures. These higher order structures can have effects on the gel physical properties (stiffness, viscosity) and may also have an effect on the reactivity of the collagen to crosslinking reagents.

Collagen concentration in an aqueous pre-reaction solution or a crosslinking reaction mixture may vary. In some embodiments, collagen may be present at a concentration of about 1 mg/mL to about 40 mg/mL, about 1 mg/mL to about 15 mg/mL, about 3 mg/mL to about 12 mg/mL, about 1.7 mg/mL, about 3 mg/mL, about 6 mg/mL, about 8 mg/mL, or about 12 mg/mL. The collagen concentration has an effect on the physical properties of the gel (stiffness, viscosity). In general, higher collagen concentrations lead to a higher elastic modulus.

In some embodiments of this disclosure, the weight ratio of hyaluronic acid to collagen in a aqueous pre-reaction solution or a aqueous pre-reaction solution or a crosslinking reaction mixture (e.g. [wt hyaluronic acid]/[wt collagen]) may be about 0.5 to about 3, about 1 to about 3, about 1 to about 2, about 1, or about 2. When the crosslinking reaction occurs, the resulting crosslinked macromolecular product may have a collagen component derived from the collagen in the crosslinking reaction. Thus, the resulting crosslinked macromolecular matrix of the present disclosure may have a weight ratio of hyaluronic acid component to collagen component that corresponds to the weight ratio in the crosslinking reaction, e.g. about 0.5 to about 3, about 1 to about 3, about 1 to about 2, about 1, or about 2. In the present invention, the weight ratio of the hyaluronic acid component to the collagen component in the crosslinked macromolecular matrix is 2.

In other embodiments of the disclosure, the compositions have an HA to collagen ratio of between about 0.5 to 1 and about 7 to 1.

A salt may help to screen the negative charges of hyaluronic acid from positive charges of collagen, and may thus prevent precipitation of a polyionic ion complex from solution. However, high concentrations of salt may reduce the solubility of some components in solution. Thus, in some embodiments, the salt concentration of an aqueous pre-reaction solution or a crosslinking reaction mixture may be high enough to screen the charges so that the polyionic ion complex is not formed, but also low enough so that the components of the mixture remain in solution. For example, the total salt concentration of some aqueous pre-reaction solutions or crosslinking reaction mixtures may be about 10 mM to about 1 M, for example, between about 5 mM to about 0.5 M, for example, between about 2 mM to about 0.2 M.

Some salts in an aqueous pre-reaction solution or a crosslinking reaction mixture may be non-coordinating buffers. Any non-coordinating buffer may be used that is capable of buffering the mixture and does not coordinate with metal atoms or ions in the collagen. In some embodiments, the buffer does not react with the crosslinking reagents (carbodiimide and additive). For example, acetate or phosphate buffers may not be used in these embodiments. Examples of suitable non-coordinating buffers may include, but are not limited to, 2-(N-morpholino)ethanesulfonic acid (MES), 3-(N-morpholino)propanesulfonic acid (MOPS), 4-(2-hydroxyethyl)-1-piperazinyl)ethanesulfonic acid (HEPES), 3-[4-(2-hydroxyethyl)-1-piperazinyl]propanesulfonic acid (HEPPS), N-cyclohexyl-2-aminoethanesulfonic acid (CHES), N-cyclohexyl-3-aminopropanesulfonic acid (CAPS), etc.

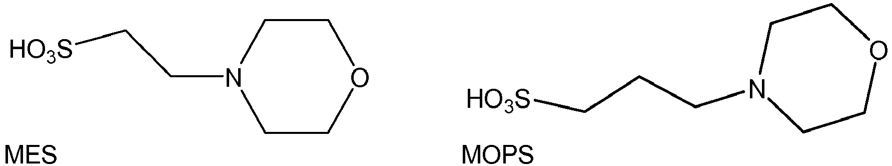

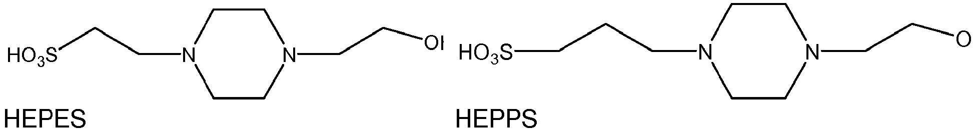

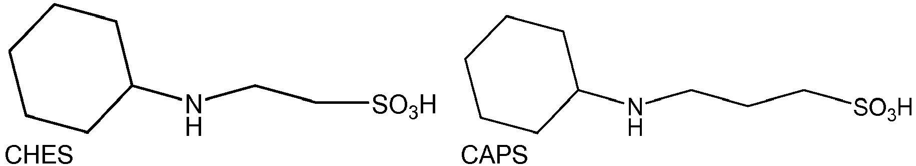

The concentration of a non-coordinating buffer may vary. For example, some aqueous pre-reaction solutions or crosslinking reaction mixtures may have a buffer concentration in a range of about 10 mM to about 1 M, about 10 mM to about 500 mM, about 20 mM to about 100 mM, or about 25 mM to about 250 mM. Some aqueous pre-reaction solutions or crosslinking reaction mixtures comprise MES at a concentration of about 20 mM to about 200 mM, about 20 mM to about 100 mM, about 100 mM, or about 180 mM.

Non-buffering salts may also be included in an aqueous pre-reaction solution or a crosslinking reaction mixture as an alternative to, or in addition, to buffering salts. Some examples may include sodium chloride, potassium chloride, potassium bromide, sodium bromide, lithium chloride, lithium bromide, sodium iodide, and potassium iodide The concentration of a non-buffering salt may vary. For example, some mixtures may have a non-buffering salt concentration in a range of about 10 mM to about 1 mM, about 30 mM to about 500 mM, or about 50 mM to about 300 mM. In some embodiments, sodium chloride may be present at a concentration in a range of about 0.5 % w/v to about 2 % about 0.9 % w/v, about 1.6 % w/v, about 20 mM to about 1 mM, about 40 mM to about 500 mM, about 50 to 300 mM, about 80 mM to about 330 mM, about 150 mM, or about 270 mM.

The pH of an aqueous pre-reaction solution may be lower than the pH of a crosslinking reaction mixture. If the salt content of the aqueous pre-reaction solution is low, the pH may be lower to enhance solubility of the hyaluronic acid and the collagen. If the salt content is higher, the pH may be higher in the aqueous pre-reaction solution. In some embodiments, the pH of the aqueous pre-reaction mixture is about 1 to about 8, about 3 to about 8, about 4 to about 6, about 4.7 to about 7.4, or about 5.4. For low salt concentrations, the pH may be about 1 to about 4 or about 1 to about 3.

In some embodiments, pH may be adjusted to neutral to allow collagen gelation or fiber formation before adding a coupling agent.

In some embodiments, the pH may be adjusted to neutral immediately prior to, around the time of, or after adding a coupling agent, such that collagen gelation is reduced or does not substantially occur.

Any water-soluble coupling agent may be used that can crosslink hyaluronic acid to collagen. Some non-limiting examples of a coupling agent include carbodiimides such as N,N'-dicyclohexylcarbodiimide (DCC), N,N'-diisopropylcarbodiimide (DIC), or 1-ethyl-3-(3-dimethylaminopropyl)carbodiimide (EDC). Carbodiimide coupling agents may facilitate ester or amide bond formation without becoming part of the linkage. However, other coupling agents that become part of the crosslinking group may be used. The concentration of a coupling agent may vary. In some embodiments, a coupling agent may be present at about 2 mM to about 150 mM, about 2 mM to about 50 mM, about 20 mM to about 100 mM, or about 50 mM. In some embodiments, the coupling agent is EDC that is present at a concentration of about 20 mM to about 100 mM, about 2 mM to about 50 mM, or about 50 mM.

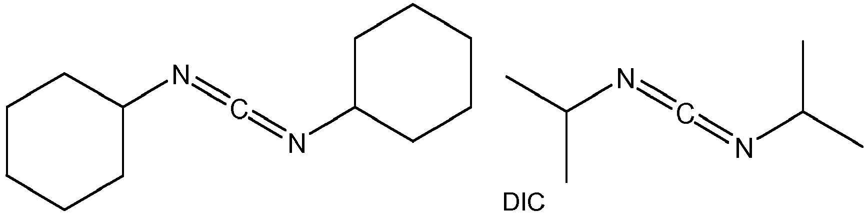

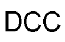

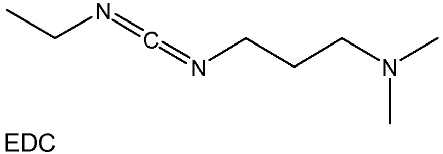

As a result of a crosslinking reaction, a crosslinked macromolecular matrix may comprise a crosslinking component that crosslinks or covalently connects the hyaluronic acid component to the collagen component. A crosslink component comprises a plurality of crosslink units, or individual covalent bonding links, between the hyaluronic acid component and the collagen component. At least a portion of the crosslink units comprise an ester bond or an amide bond. In some embodiments, at least a portion of the crosslink units may be -CON- or -CO₂-, where the N is a nitrogen from an amino acid residue.

An activating agent may be used to increase the ratio of amide bonds compared to ester bonds formed in the crosslinked product. In some embodiments, an activating agent may be a triazole such as hydroxybenzotriazole (HOBT) or 1-hydroxy-7-azabenzotriazole (HOAT); a fluorinated phenol such as pentafluorophenol; a succinimide such as N-hydroxysuccinimide (NHS) or N-hydroxysulfosuccinimide (sulfoNHS), and the like.

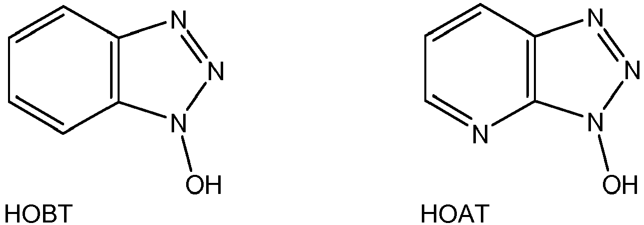

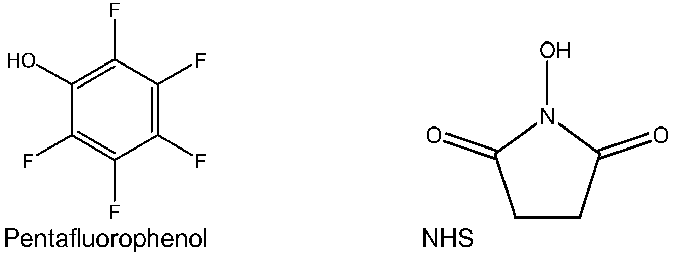

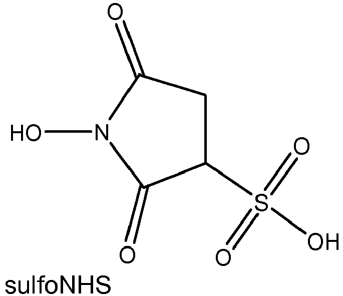

The concentration of an activating agent may vary. In some embodiments, the activating agent may have a concentration of about 2 mM to about 200 mM, about 2 mM to about 50 mM, about 20 mM to about 100 mM, or about 50 mM. In some embodments, the activating agent may be NHS or sulfoNHS is at a concentration of about 2 mM to about 50 mM. In some embodiments, the activating agent may be N-hydroxysulfosuccinimide, sodium salt, at a concentration of about 20 mM to about 100 mM, or about 50 Mm.

In some embodiments, a crosslinking reaction mixture may comprise a carbodiimide coupling agent and an activating agent. In some embodiments, the coupling agent is EDC and the activating agent is NHS or sulfoNHS. In some embodiments EDC is present at a concentration of about 2 mM to about 50 mM and NHS or sulfoNHS is present at about 2 mM to about 50 mM.

In some embodiments, a crosslinking reaction mixture may comprise hyaluronic acid at a concentration of about 1.7 mg/mL, collagen at a concentration of about 1.7 mg/mL, 2-(N-morpholino)ethanesulfonic acid at a concentration of about 100 mM, sodium chloride at a concentration of about 0.9 wt% or about 150 mM, 1-ethyl-3-(3-dimethylaminopropyl)carbodiimide at a concentration of about 50 mM, and N-hydroxysulfosuccinimide sodium salt at a concentration of about 50 Mm, wherein the solution has a pH of about 5.4.

In some embodiments, a crosslinking reaction mixture may comprise hyaluronic acid at a concentration of about 6 mg/mL, collagen at a concentration of about 6 mg/mL, 2-(N-morpholino)ethanesulfonic acid at a concentration of about 180 mM, sodium chloride at a concentration of about 1.6 wt% or about 270 mM, 1-ethyl-3-(3-dimethylaminopropyl)carbodiimide at a concentration of about 50 mM, and N-hydroxysulfosuccinimide sodium salt at a concentration of about 50 mM, wherein the solution has a pH of about 5.4.

In some embodiments, a crosslinking reaction mixture may comprise hyaluronic acid at a concentration of about 16 mg/mL of, collagen at a concentration of about 8 mg/mL, 2-(N-morpholino)ethanesulfonic acid at a concentration of about 100 mM, sodium chloride at a concentration of about 0.9 wt% or about 150 mM, 1-ethyl-3-(3-dimethylaminopropyl)carbodiimide at a concentration of about 50 mM, and N-hydroxysulfosuccinimide sodium salt at a concentration of about 50 mM, wherein the solution has a pH of between about 4.5 and 5.5, for example, about 5.2.

In some embodiments, a crosslinking reaction mixture may comprise hyaluronic acid at a concentration of about 12 mg/mL, collagen at a concentration of about 12 mg/mL, 2-(N-morpholino)ethanesulfonic acid at a concentration of about 100 mM, sodium chloride at a concentration of about 0.9 wt% or about 150 mM, 1-ethyl-3-(3-dimethylaminopropyl)carbodiimide at a concentration of about 50 mM, and N-hydroxysulfosuccinimide sodium salt at a concentration of about 50 mM.

In some embodiments, a crosslinking reaction mixture may comprise hyaluronic acid at a concentration of about 3 mg/mL, collagen at a concentration of about 3 mg/mL, 2-(N-morpholino)ethanesulfonic acid at a concentration of about 100 mM, sodium chloride at a concentration of about 0.9 wt% or about 150 mM, 1-ethyl-3-(3-dimethylaminopropyl)carbodiimide at a concentration of about 50 mM, and N-hydroxysulfosuccinimide sodium salt at a concentration of about 50 mM, wherein the solution has a pH of about 5.4.

In some embodiments, a crosslinking reaction mixture may comprise hyaluronic acid at a concentration of about 12 mg/mL, collagen at a concentration of about 6 mg/mL, 2-(N-morpholino)ethanesulfonic acid at a concentration of about 100 mM, sodium chloride at a concentration of about 0.9 wt% or about 150 mM, 1-ethyl-3-(3-dimethylaminopropyl)carbodiimide at a concentration of about 50 mM, and N-hydroxysulfosuccinimide sodium salt at a concentration of about 50 mM, wherein the solution has a pH of about 5.4.

In some embodiments, a crosslinking reaction mixture may comprise hyaluronic acid at a concentration of about 24 mg/mL, collagen at a concentration of about 12 mg/mL, 2-(N-morpholino)ethanesulfonic acid at a concentration of about 100 mM, sodium chloride at a concentration of about 0.9 wt% or about 150 mM, 1-ethyl-3-(3-dimethylaminopropyl)carbodiimide at a concentration of about 50 mM, and N-hydroxysulfosuccinimide sodium salt at a concentration of about 50 mM, wherein the solution has a pH of about 5.4.

In some embodiments, a crosslinking reaction mixture may comprise hyaluronic acid at a concentration of about 1 mg/mL to about 20 mg/mL, collagen at a concentration of about 1 mg/mL to about 15 mg/mL, 2-(N-morpholino)ethanesulfonic acid at a concentration of about 20 mM to about 200 mM, sodium chloride at a concentration of about 0.5 wt% to about 2 wt% or about 80 mM to about 330 mM, 1-ethyl-3-(3-dimethylaminopropyl)carbodiimide at a concentration of about 20 mM to about 100 mM, and N-hydroxysulfosuccinimide sodium salt at a concentration of about 20 mM to about 100 mM, wherein the solution has a pH of about 4 to about 6.

In the following, Examples 1, 2, 4, 5, 11, 12, and 13 do not form part of the invention and are provided for information purposes only.

### Example 1

### Method of making an injectable composition

Solutions of hyaluronic acid (HA) and collagen were produced by dissolving 15 mg of 2.0 MDa hyaluronic acid in 5 mL of human collagen(III) solution at 3 mg/mL in 0.01 N hydrochloric acid (Fibrogen). The hyaluronic acid / collagen solution was then lyophilized at -50°C and 0.02 Torr. The resulting sponges were soaked in 20 mL of ethanol:water mixture at ratios varying from 1:2 to 5:1 with 50 mM of 1-ethyl-3-(3-dimethylaminopropyl) carbodiimide and 50 mM of N-hydroxysulfosuccinimide sodium salt for 24 hrs. The crosslinked gels were then washed in 70% isopropanol / 30% water for sterilization followed by five washes in sterile phosphate buffer for purification.

### Example 2

### Method of making an injectable composition

A solution of HA at 3.4 mg/mL was created by dissolving 34 mg of 2 MDa HA in 10 mL of 100 mM MES buffer with 0.9 wt% NaCl, pH 4.7. Upon full hydration and dissolution of the HA, this solution was mixed with 10 mL of 3.4 mg/mL human collagen(III) solution in 100 mM HCl. The pH of the resulting HA/collagen(III) solution was adjusted to 5.4 with 10 mM NaOH solution. EDC (192 mg) and 217 mg of sulfoNHS (50 mM each) were added to the HA/collagen(III) solution and mixed thoroughly. The crosslinking reaction proceeded for 18 hrs before the gel was particulated through a 100 micron pore-sized mesh.

### Example 3

### Method of making an injectable composition

Rat tail collagen(I) in 0.01 N hydrochloric acid (Invitrogen) was concentrated from 5 mg/mL to 8 mg/mL using a centrifugal filtration device with 20 kDa molecular weight cutoff. HA (160 mg, 2 MDa) was added to 10 mL of the collagen solution and allowed to hydrate for 60 minutes. The solution was then homogenized by passing from syringe to syringe through a leur-leur connector. NaCl (93 mg) and 201 mg of MES were added to the solution and mixed. EDC (98 mg) and 111 mg of sulfoNHS were added to the solution and quickly mixed. Finally, 200 µL of 1 N NaOH was added to the solution which was mixed by syringe-to-syringe passing. The reaction solution was transferred to a glass vial and centrifuged for 5 min at 4000 RPM to remove air bubbles. The gel was then particulated through a 60 micron pore-sized mesh. Following sizing, the gel was sterilized by dialysis through a 20 kDa molecular-weight cut-off cellulose ester membrane against 70% isopropanol / 30% water for 3 hrs at 4°C. Dialysis was then continued against sterile phosphate buffer for 48 hrs at 4°C with three changes of buffer. The gel was then dispensed into syringes under aseptic conditions.

### Example 4

### Method of making an injectable composition

Rat tail collagen(I) in 0.01 N hydrochloric acid (Invitrogen) was concentrated from 5 mg/mL to 12 mg/mL using a centrifugal filtration device with 20 kDa molecular weight cutoff. HA (120 mg, 2 MDa) was added to 10 mL of the collagen solution and allowed to hydrate for 60 minutes. The solution was then homogenized by passing from syringe to syringe through a leur-leur connector. NaCl (93 mg) and 201 mg of MES were added to the solution and mixed. EDC (98 mg) and 111 mg of sulfoNHS were added to the solution and quickly mixed. Finally, 200 µL of 1 N NaOH was added to the solution which was mixed by syringe-to-syringe passing. The reaction solution was transferred to a glass vial and centrifuged for 5 min at 4000 RPM to remove air bubbles. The gel was then particulated through a 60 micron pore-sized mesh. Following sizing, the gel was sterilized by dialysis through a 20 kDa molecular-weight cut-off cellulose ester membrane against 70% isopropanol / 30% water for 3 hrs at 4°C. Dialysis was then continued against sterile phosphate buffer for 48 hrs at 4°C with three changes of buffer. The gel was then dispensed into syringes under aseptic conditions.

### Example 5

### Method of making an injectable composition

Rat tail collagen(I) in 0.01 N hydrochloric acid (Invitrogen) was concentrated from 5 mg/mL to 12 mg/mL using a centrifugal filtration device with 20 kDa molecular weight cutoff. HA (120 mg, 2 MDa) was added to 10 mL of the collagen solution and allowed to hydrate for 60 minutes. The solution was then homogenized by passing from syringe to syringe through a leur-leur connector. NaCl (93 mg), 201 mg of MES, and 200 µL of 1 N NaOH were added to the solution, mixed, and given 45 minutes for collagen polymerization. EDC (98 mg) and 111 mg of sulfoNHS were then added and the final solution was mixed by syringe-to-syringe passing. The reaction solution was transferred to a glass vial and centrifuged for 5 min at 4000 RPM to remove air bubbles. The gel was then particulated through a 60 micron pore-sized mesh. Following sizing, the gel was sterilized by dialysis through a 20 kDa molecular-weight cut-off cellulose ester membrane against 70% isopropanol / 30% water for 3 hrs at 4°C. Dialysis was then continued against sterile phosphate buffer for 48 hrs at 4°C with three changes of buffer. The gel was then dispensed into syringes under aseptic conditions.

### Example 6

### Rheology characterization of the compositions

Oscillatory parallel plate rheology was used to characterize the mechanical properties of gels using an Anton Paar MCR 301. A plate diameter of 25 mm was used at a gap height of 1 mm. A frequency sweep from 0.1 to 10 Hz at a fixed strain of 2% with logarithmic increase in frequency was applied followed by a strain sweep between 0.1% and 300% at a fixed frequency of 5 Hz with logarithmic increase in strain. The storage modulus (G') and loss modulus (G") were determined from frequency sweep measurements at 5 Hz.

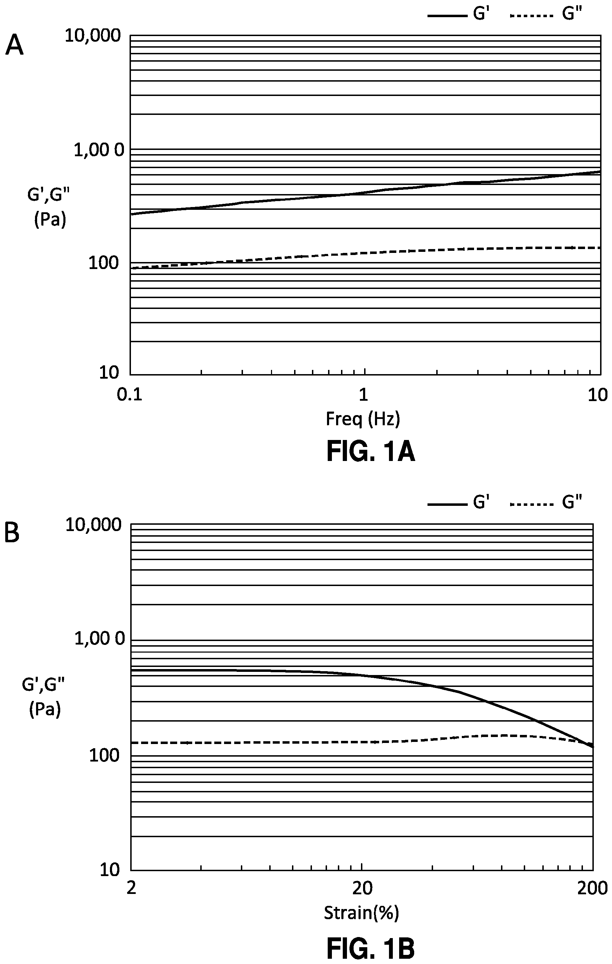

The gel from Example 4 had a storage modulus (G') of 556 Pa and loss modulus (G") of 131 Pa. The frequency sweep (A) and strain sweep (B) are shown in Figure 1.

### Example 7

### Extrusion test

In order to determine the force required to extrude the gels, they were ejected from 1 mL BD syringes through 30G needles using an Instron 5564 with Bluehill 2 software. The plunger was pushed at a rate of 100 mm/min for 11.35 mm and the extrusion profile was recorded.

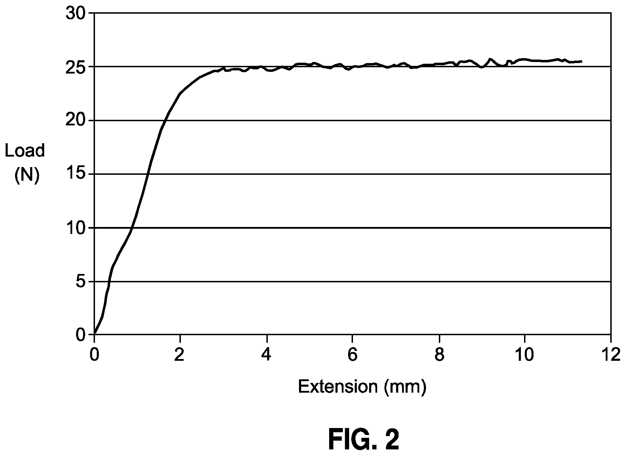

The extrusion profile through a 30G needle for gel from Example 4 is shown in Figure 2. The gel had an average extrusion force of 25 N from 4 through 10 mm.

### Example 8

### Method of making dermal fillers

Hyaluronic acid, 2 MDa molecular weight, was dissolved in human collagen(I) solution in 0.01 N hydrochloric acid (Advanced BioMatrix). Sodium chloride was added at 0.9 wt% and 2-(N-morpholino)ethanesulfonic acid was added at 100 mM to the solution and mixed. The hyaluronic acid was allowed to hydrate for 1 hr and the solution was homogenized by syringe-to-syringe mixing. The pH of the solution was adjusted to 5.4 by addition of 1 N sodium hydroxide. 1-Ethyl-3-(3-dimethylaminopropyl) carbodiimide (50 mM) and N-hydroxysulfosuccinimide sodium salt (50 mM) were added to the hyaluronic acid / collagen solution and quickly mixed by syringe-to-syringe transfer. The solution was transferred to a glass vial and centrifuged for 5 min at 4000 RPM to remove air bubbles. The resulting gel was allowed to react for 16 hrs at 4 °C. The gel was then particulated through a 100 micron pore-sized mesh. Following sizing, the gel was sterilized by dialysis through a 20 kDa molecular-weight cut-off cellulose ester membrane against 70% isopropanol / 30% water for 3 hrs at 4°C. Dialysis was then continued against sterile phosphate buffer, pH 7.4, for 48 hrs at 4°C with four changes of buffer. The gel was then dispensed into syringes under aseptic conditions.

This procedure was used to produce hydrogels with varying concentrations of hyaluronic acid and collagen. When required, human collagen(I) in 0.01 N hydrochloric acid was concentrated from 3 mg/mL to the desired reaction concentration in 20 kDa molecular-weight cut-off centrifugal filtration devices. A 50 mL sample of each gel was synthesized, sterilized by exposure to 70% isopropanol, and purified by dialysis against phosphate buffer, pH 7.4. The gels synthesized are described in Table 2 along with their rheological properties.

**Table 2: Hyaluronic acid-human collagen(I) hydrogel synthesis concentrations and rheological properties.**

| **Sample ID** | **[HA] (mg/mL)** | **[Col(I)] (mg/mL)** | **G' (Pa)** | **G" (Pa)** |
|---|---|---|---|---|
| **A*** | 3 | 3 | 199 | 24.6 |
| **B** | 12 | 6 | 1260 | 154 |
| **C** | 16 | 8 | 2450 | 288 |
| **D*** | 12 | 12 | 3160 | 420 |
| **E** | 24 | 12 | 5440 | 433 |
| **F*** | 12 | 3 | 1110 | 52.2 |
| **G*** | 16 | 3 | 1490 | 60.6 |
| **H*** | 20 | 3 | 1770 | 49.5 |

| | | | | |
|---|---|---|---|---|
| *These examples do not form part of the invention and are provided for information purposes only. | | | | |

### Example 9

### Biopolymer concentration of the dermal fillers

In order to determine the biopolymer concentration in gels, the weight of the hydrated gel was compared to that of dried gel. A 2 mL sample of gel was weighed and dried by flash-freezing in liquid nitrogen followed by lyophilization at -50°C and 0.02 Torr. A solution of the appropriate buffer was also weighed and dried in the same fashion to account for salt content of the gel. The total solids content of the gel was calculated by dividing the dry weight by the wet volume, assuming 1 g/mL density for the wet gel, to give a value in mg/mL. The salt solids content was then subtracted from this value to determine the biopolymer concentration in the gel.

**Table 3: Final concentrations of hyaluronic acid-human collagen(I) hydrogels.**

| Sample ID | [HA](mg/mL) | [Col(I)] (mg/mL) | Final concentration (mg/mL) |
|---|---|---|---|
| A* | 3 | 3 | 5.3 |
| B | 12 | 6 | 16.3 |
| C | 16 | 8 | 19.4 |
| D* | 12 | 12 | 22.6 |
| E | 24 | 12 | 31.6 |

| | | | |
|---|---|---|---|
| *These examples do not form part of the invention and are provided for information purposes only. | | | |

### Example 10

### Swelling ratios

Swelling ratios relative to initial water content were determined for gels by increase in weight when equilibrated with phosphate buffer. For each gel, approximately 1 mL was injected into a 15 mL Falcon tube and weighed, followed by addition of 10 mL of phosphate buffered saline, pH 7.4. The gels were thoroughly mixed with the buffer and vortexed for 30 seconds. The gels were then allowed to equilibrate in the buffer for 48 hrs at 4°C. After this time, the suspensions were centrifuged at 4000 RPM in a swinging bucket rotor for 5 minutes. The supernatant buffer was then decanted and the weight of the swollen gel was measured. The swelling ratio was determined by dividing the final weight of the swollen gel by the weight of the initial gel.

**Table 4: Swelling ratios of hyaluronic acid-human collagen(I) hydrogels.**

| Sample ID | [HA] (mg/mL) | [Col(I)] (mg/mL) | Swelling ratio |
|---|---|---|---|
| A* | 3 | 3 | 0.96 |
| B | 12 | 6 | 1.67 |
| C | 16 | 8 | 1.69 |
| D* | 12 | 12 | 1.49 |
| E | 24 | 12 | 1.65 |

| | | | |
|---|---|---|---|
| *These examples do not form part of the invention and are provided for information purposes only. | | | |

### Example 11

### HA/Collagen for Facial Defects of Check

This example illustrates the use of compositions and methods disclosed herein for a facial disorder.

A 58-year-old woman presented with a lean face. She felt her face looked old, sad and bitter because of the less fullness of her cheek contour. Pre-operative evaluation of the person includes routine history and physical examination in addition to thorough informed consent disclosing all relevant risks and benefits of the procedure. The physician evaluating the individual determines that she is a candidate for administration of the dermal filler compositions and methods disclosed herein.

A composition of the invention, such as described in EXAMPLE 4, is provided in a 20 mL syringe. One-holed blunt infiltration cannulas (3 mm inner diameter) are used to place about 15 mL of the composition in the syringe subcutaneously and under superficial musculoaponeurotix system into the left and right checks.

The individual is monitored for approximately 7 days. The physician evaluates the treatment area and determines that the treatment was successful. The woman's cheeks are fuller than prior to treatment, Both the woman and her physician are satisfied with the results of the procedure because she looks younger than she did when she came in for treatment.

### Example 12

### Treatment of Facial Defects of Eyelids

This example illustrates the use of compositions and methods disclosed herein for a treatment of eyelid defects.

A 37-year-old woman presented with fine wrinkles around her eyes and she reports that her eyes made her look old and angry. Pre-operative evaluation of the person includes routine history and physical examination in addition to thorough informed consent disclosing all relevant risks and benefits of the procedure. The physician evaluating the individual determines that she is a candidate for administration of the dermal filler compositions and methods disclosed herein.

A composition, such as made as described in Example 5, is provided in a 20 mL syringe. About 2.5 mL of the composition is injected with a fine needle subcutaneously in the skin beneath the wrinkles into the regions adjacent the eyes.

The individual is monitored for approximately 7 days. The physician evaluates the eye of the patient and determines that the treatment was successful. Both the woman and her physician are satisfied with the results of the procedure because her eyes appear refreshed and the skin appears rejuvenated. Approximately one year after the procedure, the woman indicates that her quality of life has improved.

### Example 13

### Treatment of acne scars

This example illustrates the use of compositions and methods disclosed herein for treatment of acne scars.

A 25-year-old man presents with moderate acne scarring on his jaw line including depressions and pitting. He reports that he is dissatisfied with his appearance and feels he is socially inhibited due to his perception of his appearance. Pre-operative evaluation of the person includes routine history and physical examination in addition to thorough informed consent disclosing all relevant risks and benefits of the procedure. The physician evaluating the individual determines that he is a candidate for administration of the dermal filler compositions and methods disclosed herein.

A composition, such as that made as described in Example 12, is provided in 10 mL syringes. The physician injects a small amount of the composition below the skin in each depressed or pitted area of the patient's jawline to raise the area to match the surrounding skin.

The individual returns for a follow up visit with the physician in 14 days. The physician evaluates the patient and determines that the treatment was successful. The man reports he is satisfied with the results of the procedure because his skin is more smooth in appearance and the acne scarring is substantially less visible. Approximately six months after the procedure, the man returns for a follow up treatment. He reports to the physician that his quality of life has greatly improved since the procedure and he is no longer shy about his appearance.

Unless otherwise indicated, all numbers expressing quantities of ingredients, properties such as molecular weight, reaction conditions, and so forth used in the specification and claims are to be understood as being modified in all instances by the term "about." Accordingly, unless indicated to the contrary, the numerical parameters set forth in the specification and attached claims are approximations that may vary depending upon the desired properties sought to be obtained. At the very least, and not as an attempt to limit the application of the doctrine of equivalents to the scope of the claims, each numerical parameter should at least be construed in light of the number of reported significant digits and by applying ordinary rounding techniques.

The terms "a," "an," "the," and similar referents used in the context of describing the invention (especially in the context of the following claims) are to be construed to cover both the singular and the plural, unless otherwise indicated herein or clearly contradicted by context. All methods described herein can be performed in any suitable order unless otherwise indicated herein or otherwise clearly contradicted by context. The use of any and all examples, or exemplary language (e.g., "such as") provided herein is intended merely to better illuminate the invention and does not pose a limitation on the scope of any claim. No language in the specification should be construed as indicating any non-claimed element essential to the practice of the invention.

Groupings of alternative elements or embodiments disclosed herein are not to be construed as limitations. Each group member may be referred to and claimed individually or in any combination with other members of the group or other elements found herein. It is anticipated that one or more members of a group may be included in, or deleted from, a group for reasons of convenience and/or patentability. When any such inclusion or deletion occurs, the specification is deemed to contain the group as modified thus fulfilling the written description of all Markush groups used in the appended claims.

Certain embodiments are described herein, including the best mode known to the inventors for carrying out the invention. Of course, variations on these described embodiments will become apparent to those of ordinary skill in the art upon reading the foregoing description. The inventor expects skilled artisans to employ such variations as appropriate, and the inventors intend for the invention to be practiced otherwise than specifically described herein. Accordingly, the claims include all modifications and equivalents of the subject matter recited in the claims as permitted by applicable law. Moreover, any combination of the above-described elements in all possible variations thereof is contemplated unless otherwise indicated herein or otherwise clearly contradicted by context.

In closing, it is to be understood that the embodiments disclosed herein are illustrative of the principles of the claims. Other modifications that may be employed are within the scope of the claims. Thus, by way of example, but not of limitation, alternative embodiments may be utilized in accordance with the teachings herein. Accordingly, the claims are not limited to embodiments precisely as shown and described.

## Claims

1. A soft tissue aesthetic product comprising:
a filler comprising a hydrogel having a form suitable for injecting into mammalian tissue; and
a label comprising instructions to inject the filler into the tissue;
wherein the hydrogel comprises water, and a crosslinked macromolecular matrix comprising:
a hyaluronic acid component; and
a collagen component;
wherein the hyaluronic acid component is crosslinked to the collagen component by a crosslinking component;
wherein the crosslinking component comprises a plurality of crosslink units, wherein at least a portion of the crosslink units comprise an ester bond or an amide bond,and
wherein the weight ratio of the hyaluronic acid component to the collagen component in the crosslinked macromolecular matrix is 2.

2. The product of claim 1, wherein the collagen component comprises collagen type I or collagen type III.

3. The product of claim 1, wherein the hydrogel is in the form of a dermal filler.

4. The product of claim 1, wherein the hydrogel is in the form of a lip filler.

5. A method of improving an aesthetic quality of soft tissue of a human being comprising:
injecting a hydrogel composition into a soft tissue of the human being to thereby improve the aesthetic quality of the soft tissue;
wherein the hydrogel composition comprises water, and a crosslinked macromolecular matrix comprising:
a hyaluronic acid component; and
a collagen component;
wherein the hyaluronic acid component is crosslinked to the collagen component by a crosslinking component; and
wherein the crosslinking component comprises a plurality of crosslink units, wherein at least a portion of the crosslink units comprise an ester bond or an amide bond,and
wherein the weight ratio of the hyaluronic acid component to the collagen component in the crosslinked macromolecular matrix is 2.

6. The method of claim 5, wherein the collagen component comprises collagen type I or collagen type III.

7. The method of claim 5, wherein the hydrogel is in the form of a dermal filler or a lip filler.

8. A dermal filler comprising:
a hydrogel having a form suitable for injecting into mammalian tissue;
wherein the hydrogel comprises water, and a crosslinked macromolecular matrix comprising:
a hyaluronic acid component; and
a collagen component;
wherein the hyaluronic acid component is crosslinked to the collagen component by a crosslinking component;
wherein the crosslinking component comprises a plurality of crosslink units, wherein at least a portion of the crosslink units comprise an ester bond or an amide bond,and
wherein the weight ratio of the hyaluronic acid component to the collagen component in the crosslinked macromolecular matrix is 2.

9. The dermal filler of claim 8, wherein the collagen component comprises collagen type I or collagen type III.

## Patentansprüche

1. Weichgewebe-Ästhetikprodukt, umfassend:
einen Filler, der ein Hydrogel umfasst, das eine zum Injizieren in Säugergewebe geeignete Form aufweist; und
ein Etikett, das Anweisungen zum Injizieren des Fillers in das Gewebe umfasst;
wobei das Hydrogel Wasser und eine vernetzte makromolekulare Matrix umfasst, die umfasst:
eine Hyaluronsäurekomponente; und
eine Kollagenkomponente;
wobei die Hyaluronsäurekomponente mit der Kollagenkomponente mittels einer vernetzenden Komponente vernetzt ist;
wobei die vernetzende Komponente eine Mehrzahl an Vernetzungseinheiten umfasst, wobei wenigstens ein Abschnitt der Vernetzungseinheiten eine Esterbindung oder eine Amidbindung umfasst und
wobei das Gewichtsverhältnis der Hyaluronsäurekomponente zu der Kollagenkomponente in der vernetzten makromolekularen Matrix 2 beträgt.

2. Produkt gemäß Anspruch 1, wobei die Kollagenkomponente Kollagen Typ I oder Kollagen Typ III umfasst.

3. Produkt gemäß Anspruch 1, wobei das Hydrogel in der Form eines Dermalfillers ist.

4. Produkt gemäß Anspruch 1, wobei das Hydrogel in der Form eines Lippenfillers ist.

5. Verfahren zur Verbesserung einer ästhetischen Eigenschaft eines Weichgewebes eines menschlichen Wesens, umfassend:
Injizieren einer Hydrogelzusammensetzung in ein Weichgewebe des menschlichen Wesens, wodurch die ästhetische Eigenschaft des Weichgewebes verbessert wird;
wobei die Hydrogelzusammensetzung Wasser und eine vernetzte makromolekulare Matrix umfasst, die umfasst:
eine Hyaluronsäurekomponente; und
eine Kollagenkomponente;
wobei die Hyaluronsäurekomponente mit der Kollagenkomponente mittels einer vernetzenden Komponente vernetzt ist;
wobei die vernetzende Komponente eine Mehrzahl an Vernetzungseinheiten umfasst, wobei wenigstens ein Abschnitt der Vernetzungseinheiten eine Esterbindung oder eine Amidbindung umfasst und
wobei das Gewichtsverhältnis der Hyaluronsäurekomponente zu der Kollagenkomponente in der vernetzten makromolekularen Matrix 2 beträgt.

6. Verfahren gemäß Anspruch 5, wobei die Kollagenkomponente Kollagen Typ I oder Kollagen Typ III umfasst.

7. Verfahren gemäß Anspruch 5, wobei das Hydrogel in der Form eines Dermalfillers oder eines Lippenfillers ist.

8. Dermalfiller, umfassend:
ein Hydrogel, das eine zum Injizieren in Säugergewebe geeignete Form aufweist;
wobei das Hydrogel Wasser und eine vernetzte makromolekulare Matrix umfasst, die umfasst:
eine Hyaluronsäurekomponente; und
eine Kollagenkomponente;
wobei die Hyaluronsäurekomponente mit der Kollagenkomponente mittels einer vernetzenden Komponente vernetzt ist;
wobei die vernetzende Komponente eine Mehrzahl an Vernetzungseinheiten umfasst, wobei wenigstens ein Abschnitt der Vernetzungseinheiten eine Esterbindung oder eine Amidbindung umfasst und
wobei das Gewichtsverhältnis der Hyaluronsäurekomponente zu der Kollagenkomponente in der vernetzten makromolekularen Matrix 2 beträgt.

9. Dermalfiller gemäß Anspruch 8, wobei die Kollagenkomponente Kollagen Typ I oder Kollagen Typ III umfasst.

## Revendications

1. Produit d'esthétique pour tissu mou comprenant :
un agent de comblement comprenant un hydrogel ayant une forme appropriée pour injection dans un tissu de mammifère; et
une étiquette comprenant des instructions pour injecter l'agent de comblement dans le tissu ;
dans lequel l'hydrogel comprend de l'eau, et une matrice macromoléculaire réticulée comprenant :
un composant d'acide hyaluronique ; et
un composant de collagène ;
dans lequel le composant d'acide hyaluronique est réticulé au composant de collagène par un composant de réticulation ;
dans lequel le composant de réticulation comprend une pluralité d'unités de réticulation, dans lesquelles au moins une partie des unités de réticulation comprend une liaison ester ou une liaison amide ; et
dans lequel le rapport pondéral du composant d'acide hyaluronique au composant de collagène dans la matrice macromoléculaire réticulée est 2.

2. Produit selon la revendication 1, dans lequel le composant de collagène comprend du collagène de type I ou du collagène de type III.

3. Produit selon la revendication 1, dans lequel l'hydrogel est sous la forme d'un agent de comblement dermique.

4. Produit selon la revendication 1, dans lequel l'hydrogel est sous la forme d'un agent de comblement pour les lèvres.

5. Procédé d'amélioration d'une qualité esthétique d'un tissu mou d'un être humain comprenant :
l'injection d'une composition d'hydrogel dans un tissu mou de l'être humain pour améliorer ainsi la qualité esthétique du tissu mou ;
dans lequel la composition d'hydrogel comprend de l'eau, et une matrice macromoléculaire réticulée comprenant :
un composant d'acide hyaluronique ; et
un composant de collagène ;
dans lequel le composant d'acide hyaluronique est réticulé au composant de collagène par un composant de réticulation ; et
dans lequel le composant de réticulation comprend une pluralité d'unités de réticulation, dans lequel au moins une partie des unités de réticulation comprend une liaison ester ou une liaison amide, et
dans lequel le rapport pondéral du composant d'acide hyaluronique au composant de collagène dans la matrice macromoléculaire réticulée est 2.

6. Procédé selon la revendication 5, dans lequel le composant de collagène comprend du collagène de type I ou du collagène de type III.

7. Procédé selon la revendication 5, dans lequel l'hydrogel est sous la forme d'un agent de comblement dermique ou d'un agent de comblement pour lèvres.

8. Agent de comblement dermique comprenant :
un hydrogel ayant une forme appropriée pour injection dans un tissu de mammifère ;
dans lequel l'hydrogel comprend de l'eau, et une matrice macromoléculaire réticulée comprenant :
un composant d'acide hyaluronique ; et
un composant de collagène ;
dans lequel le composant d'acide hyaluronique est réticulé au composant de collagène par un composant de réticulation ; et
dans lequel le composant de réticulation comprend une pluralité d'unités de réticulation, dans lequel au moins une partie des unités de réticulation comprend une liaison ester ou une liaison amide, et
dans lequel le rapport pondéral du composant d'acide hyaluronique au composant de collagène dans la matrice macromoléculaire réticulée est 2.

9. Agent de comblement dermique selon la revendication 8, dans lequel le composant de collagène comprend du collagène de type I ou du collagène de type III.
